# EUROPÄISCHE PATENTSCHRIFT

(11) **EP 0 803 273 B1**
(45) Veröffentlichungstag und Bekanntmachung des Hinweises auf die Patenterteilung: **17.07.2002**
(21) Anmeldenummer: 97106775.6
(22) Anmeldetag: 24.04.1997
(51) Int. Cl.: B01D 19/00, A61M 1/36

(54) **Vorrichtung zum Abscheiden von Gasblasen aus Flüssigkeiten, insbesondere Blut**
Device for separating gas bubbles from liquids, particularly blood
Dispositif de séparation de bulles de gaz d'un liquide, particulièrement du sang

(30) Priorität: 27.04.1996 DE 19617036
(43) Veröffentlichungstag der Anmeldung: 29.10.1997
(73) Patentinhaber: Fresenius Medical Care Deutschland GmbH, 61346 Bad Homburg (DE)
(72) Erfinder: Wojke, Ralf, Dr., 61352 Bad Homburg v.d.H. (DE)
(74) Vertreter: Luderschmidt, Schüler & Partner GbR

(56) Entgegenhaltungen:
- EP-A- 0 161 803
- EP-A- 0 676 213
- EP-A- 0 728 509
- DE-A- 3 543 126
- DE-A- 4 329 385
- GB-A- 2 041 233

## Beschreibung

Die Erfindung betrifft eine Vorrichtung zum Abscheiden von Gasblasen aus Flüssigkeiten, insbesondere Blut.

Wenn Blut dem natürlichen Blutkreislauf eines Patienten entnommen und durch einen künstlichen Blutkreislauf geleitet wird, ist es erforderlich, eventuell im Blut enthaltene Luftblasen abzuscheiden, bevor das Blut wieder dem Körper des Patienten zugeführt wird. Die Abscheidung von Luftblasen aus Blut ist beispielsweise bei der Zellseparation im Rahmen der Autotransfusion von Blut während einer Operation sowie bei der Hämodialyse oder Hämofiltration erforderlich.

Die bekannten Vorrichtungen zum Abscheiden von Luftblasen aus medizinischen Flüssigkeiten, z.B. Blut, können auch zum Abscheiden von anderen Gasen als Luft Verwendung finden. Daher werden derartige Luftabscheider auch als Entgasungsvorrichtung bezeichnet.

Insbesondere bei der Entgasung von Blut stellt sich das Problem, daß die Abscheidung von Luftblasen einerseits mit großer Zuverlässigkeit erfolgen muß und der Luftabscheider andererseits hinsichtlich seiner mechanischen Eigenschaften und der sich ausbildenden Strömungsbahnen so beschaffen sein muß, daß Beschädigungen der Blutbestandteile ausgeschlossen werden.

Der aus GB 2063108 A bekannte Luftabscheider weist eine senkrecht angeordnete Kammer mit einem zylindrischen Abschnitt auf, der in einen kegelförmigen Abschnitt übergeht. An der Spitze des kegelförmigen Kopfstückes der Kammer ist eine Entlüftungsbohrung vorgesehen. Die zu entgasende Flüssigkeit tritt unterhalb des kegelförmigen Abschnitts in die Kammer ein. Der Einlaufstutzen ist derart angeordnet, daß die Flüssigkeit im Bereich des äußeren Umfangs tangential in die Kammer strömt. Aufgrund der tangentialen Einleitung fließt die Flüssigkeit zunächst auf einer kreisförmigen Strömungsbahn, die jedoch von der gesamten Srömung durch die Kammer überlagert wird, so daß die Flüssigkeit die Kammer in einer schraubenlinienförmigen Strömungsbahn durchströmt und am unteren Ende der Kammer aus dem tangential angeordneten Auslaßstutzen wieder aus der Kammer austritt. Die kreisförmigen Bewegungsanteile der Flüssigkeitsströmung erzeugen dabei Zentrifugalkräfte, die in der Flüssigkeit Druckunterschiede aufbauen, so daß die Luftblasen zur Mitte der Kammer gedrängt werden und nach oben aufsteigen. Die abgeschiedenen Luftblasen können dann durch die Entlüftungsbohrung am oberen Ende der Kammer abgeleitet werden.

DE 4329385 A1 beschreibt einen Luftabscheider mit einem stromab des Einlaßstutzens befindlichen Strömungsleitbauteil, das zwei im Abstand zueinander angeordnete Rotationskörper aufweist, zwischen denen sich Leitschaufeln erstrecken. Die Leitschaufeln begrenzen Strömungskanäle, die sich in einer Raumkurve aus der Kammerlängsrichtung in eine im wesentlichen tangential zur Wandung der Kammer verlaufende Richtung erstrecken. Die aufsteigenden Luftblasen bilden im oberen Teil der Kammer ein Luftpolster. Die Flüssigkeitsaustrittsöffnungen des Strömungsleitbauteils sind in ausreichendem Abstand zu dem Deckelteil der Kammer angeordnet, damit die Öffnungen unterhalb des sich in der Kammer ausbildenden Flüssigkeitsspiegels liegen.

Bei der Atmung liegen die Kontaktzeiten zwischen den Blutbestandteilen und der Luft unterhalb einer Sekunde. Bei längeren Kontaktzeiten werden unterschiedliche Regelmechanismen z.B. die Blutgerinnung in Gang gesetzt. Im extrakorporalen Kreislauf kann der Blut-Luft-Kontakt jedoch zu Komplikationen bis hin zum Behandlungsabbruch führen. Insofern erweist sich das bei den bekannten Luftabscheidern im oberen Teil der Kammer eingeschlossenes Luftvolumen als nachteilig.

Der Erfindung liegt die Aufgabe zugrunde, eine Vorrichtung zum Abscheiden von Gasblasen aus Flüssigkeiten, insbesondere Blut, zu schaffen, mit der sich ein Blut-Luft-Kontakt vermeiden läßt und die eine Abscheidung von Luft auch nach mehrstündigem Gebrauch noch mit großer Sicherheit erlaubt.

Die Lösung der Aufgabe erfolgt erfindungsgemäß mit den Merkmalen des Patentanspruchs 1.

Bei der erfindungsgemäßen Vorrichtung ist im Deckelteil der Kammer, d.h. am höchsten Punkt der Kammer, mindestens eine mit einer hydrophoben Membran verschlossene Öffnung vorgesehen. Die hyrophobe Membran läßt Gase durch und hält Flüssigkeit zurück. Beim Befüllen der Kammer entweicht die in der Kammer eingeschlossene Luft über die hyrophobe Membran. Unter der Voraussetzung, daß der Systemdruck oberhalb des Atmosphärendrucks liegt, kann die Kammer vollständig mit Flüssigkeit gefüllt werden. Derartige Druckverhältnisse liegen z.B. dann vor, wenn die erfindungsgemäße Vorrichtung bei der Dialysebehandlung in die arterielle Blutleitung stromab der Blutpumpe oder in die venöse Blutleitung geschaltet ist. Dieser Überdruck wird durch die im extrakorporalen Kreislauf angeordnete Blutpumpe erzeugt, um einen hinreichend großen Fluß im System zu erzeugen und wird, beispielsweise im venösen Zweig des extrakorporalen Kreislaufs, aus Sicherheitsgründen standardmäßig überwacht. Eine Druckumkehr kann nicht auftreten, da bei Unterschreiten des unteren Grenzwertes Alarm gegeben und das Dialysegerät in den sicheren Zustand überführt wird.

Bei der erfindungsgemäßen Vorrichtung ist die Strömung in der Kammer so gestaltet, daß keine Totzonen auftreten und die Membran im Deckelteil vollständig umspült wird. Hierzu ist der im wesentlichen tangential zur Wandung der Kammer verlaufende Abschnitt des mindestens einen Strömungskanals unmittelbar unterhalb des Deckelteils angeordnet. Bei einer derartigen Anordnung bildet sich eine starke Strömung entlang der im Deckelteil befindlichen Membran aus. Die radiale und rotierende Strömungsführung reißt sich eventuell unterhalb des Deckelteils festsetzende Luftblasen mit, so daß diese durch die Membran austreten können. Da die Luftblasen mitgerissen werden, braucht sich die Membran nicht über die gesamte Oberseite der Kammer zu erstrecken. Die Membran kann auch nur ein Teil der Fläche des Deckelteils einnehmen, was aus Kostengründen von Vorteil ist. Selbst wenn die Kammer nicht exakt positioniert sein sollte und sich der Teil der Deckelfläche ohne Membran oberhalb des Teils der Deckelfläche mit Membran befinden sollte, kann sich aufgrund der besonderen Strömungsführung ein Luftpolster nicht dauerhaft ausbilden.

Ferner hat sich gezeigt, daß die Luftdurchlässigkeit einer von Blut umspülten Membran im Laufe der Zeit nur unwesentlich abnimmt, während eine Membran, die nicht von Blut umspült wird, nach längerem Blutkontakt ihre Luftdurchlässigkeit verliert. Da das Auftreten von Totzonen vermieden wird, erlaubt die erfindungsgemäße Vorrichtung eine Abscheidung von Gasblasen aus medizinischen Flüssigkeiten mit großer Sicherheit noch nach mehrstündigem Gebrauch. Bei der erfindungsgemäßen Vorrichtung werden eine vollständige Füllung, lange Standzeiten und eine hohe Luftdurchlässigkeit bei Blutkontakt erreicht. Es wird keine Luft eingeschlossen und der unphysiologische Blut-Luft-Kontakt unterbleibt. Während der Einlauf der Flüssigkeit bei den bekannten Kammern, in denen sich im oberen Kammerbereich ein Luftpolster ausbildet, möglichst tief gelegt wird, um die Flüssigkeitsschicht zu beruhigen und eine erneute Zufuhr von Luft zu vermeiden, was zu einer relativ großen Bauhöhe führt, kann die Bauhöhe der Kammer der erfindungsgemäßen Vorrichtung relativ niedrig gehalten werden, wodurch sich Material einsparen läßt.

In einer bevorzugten Ausführungsform weist das Strömungsleitbauteil ein zentrales rohrförmiges Anschlußstück auf, daß an dem Einlaufstutzen angeschlossen ist. Das zentrale Anschlußstück kann in den Einlaufstutzen passend eingesetzt sein. Es ist aber auch möglich, daß das Anschlußstück einstückiger Bestandteil des Einlaufstutzens ist. Das Anschlußstück des Strömungsleitbauteils geht in mindestens ein Strömungsleitrohr über, das sich in einer Raumkurve aus der Kammerlängsrichtung in eine im wesentlichen tangential zur Wandung der Kammer verlaufende Richtung erstreckt. Wesentlich ist, daß das Anschlußstück kurz ist, so daß die Austrittsöffnungen der Strömungsleitrohre dicht unterhalb der Unterseite des Deckelteils liegen.

Mit den Strömungsleitrohren ermöglicht das Strömungsleitbauteil eine kontinuierliche, Stöße weitgehend vermeidende Strömungsführung, so daß in der zu entgasenden Flüssigkeit ein gleichmäßiges Strömungsprofil erzeugt wird. Dies führt zu niedrigen und gleichmäßigen Schubspannungen, so daß das Abscheiden von Luftblasen aus Luft mit einer minimalen Blutschädigung verbunden ist.

Das Strömungsleitbauteil weist vorzugsweise 2 Strömungsleitrohre auf, die derart angeordnet sind, daß deren Öffnungen in entgegengesetzte Richtungen weisen. Dadurch wird erreicht, daß sich die beiden Strömungen nicht überlagern und Verwirbelung im Bereich des Strömungsbauteils vermieden werden.

Die mit der hydrophoben Membran verschlosssene Öffnung im Deckelteil ist vorzugsweise kreisförmig. Sie kann aber auch rechteckförmig, oval oder kreisringförmig ausgebildet sein. Im Sinne einer verbesserten Luftabscheidung sollte sich die neben dem zentralen Einlaufstutzen angeordnete kreisförmige Öffnung über die gesamte zur Verfügung stehende Breite des Deckelteils, d.h. zwischen dem zentralen Einlaufstutzen und der Wandung der Kammer erstrecken. Anstelle einer Öffnung können im Deckelteil auch mehrere Öffnungen zwischen dem zentralen Einlaufstutzen und der Wandung der Kammer umfangsmäßig verteilt angeordnet sein.

In einer weiteren bevorzugten Ausführungsform der Erfindung weist das Strömungsleitbauteil einen rotationssymetrischen Grundkörper auf, dessen der einströmenden Flüssigkeit zugewandte Oberfläche mit Leitschaufeln versehen ist, die in auf der Rotationsachse senkrecht stehenden Ebenen gekrümmt sind. Die durch die Leitschaufeln seitlich begrenzten Strömungskanäle geben schraublinienförmige Strömungsbahnen vor. Die kreisförmigen Bewegungsanteile der Strömung sorgen einerseits für eine optimale Umspülung der Membran und führen andererseits zu einer verbesserten Luftabscheidung.

Das Strömungsleitbauteil weist vorzugsweise einen zweiten rotationssymmetrischen Körper auf, der vorteilhafterweise unter Ausbildung von mehreren an seiner Oberseite geschlossenen Strömungskanälen im Abstand stromauf des Grundkörpers angeordnet ist. Dadurch wird die Strömung genau geführt und Strömungsablösungen sowie die Induzierung von Wirbeln etc. können weitgehend verhindert werden, was unter dem Gesichtspunkt einer minimalen Blutschädigung von Vorteil ist.

Im folgenden werden unter Bezugnahme auf die Zeichnungen zwei Ausführungsbeispiele der Erfindung näher erläutert.

Es zeigen:
- Figur 1: einen Längsschnitt durch eine erste Ausführungsform des Luftabscheiders,
- Figur 2: den Deckel der Kammer mit dem Strömungsleitbauteil in teilweise geschnittener vergrößerter Darstellung,
- Figur 3: eine Ansicht des Deckels der Kammer von Figur 2 aus der Richtung des Pfeils III,
- Figur 4: das aus zwei Teilstücken bestehende Strömungsleitbauteil des Luftabscheiders in vergrößerter Darstellung,
- Figur 5: das obere und untere Teilstück des Strömungsleitbauteils in der Seitenansicht vor dem Zusammensetzen,
- Figur 6: eine Ansicht des unteren Teilstücks des Strömungsleitbauteils aus der Richtung des Pfeils VI von Figur 5,
- Figur 7: das untere Teilstück des Strömungsleitbauteils von Figur 6 in der Draufsicht,
- Figur 8: eine zweite Ausführungsform des an den Einlaufstutzen der Kammer angeschlossenen Strömungsleitbauteils in vergrößerter perspektivischer Darstellung und
- Figur 9: eine Draufsicht auf den Grundkörper des Strömungsleitbauteils von Figur 8.

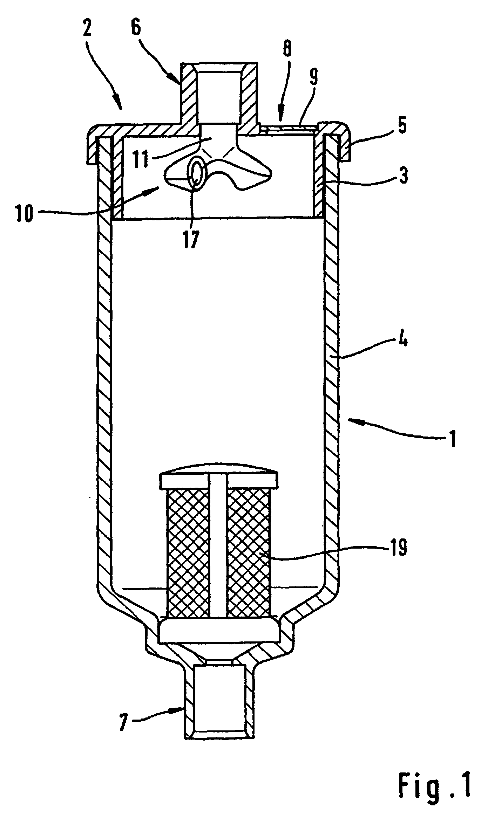

Figur 1 zeigt den senkrecht angeordneten Luftabscheider in teilweise geschnittener Darstellung. Der Luftabscheider weist eine im wesentlichen kreiszylinderförmige Kammer 1 auf, die an ihrem oberen Ende mit einem Deckel 2 verschlossen ist, der mit einem zylindrischen Flansch 3 und einem umlaufenden die Wand 4 der Kammer 1 übergreifenden Rand 5 versehen ist.

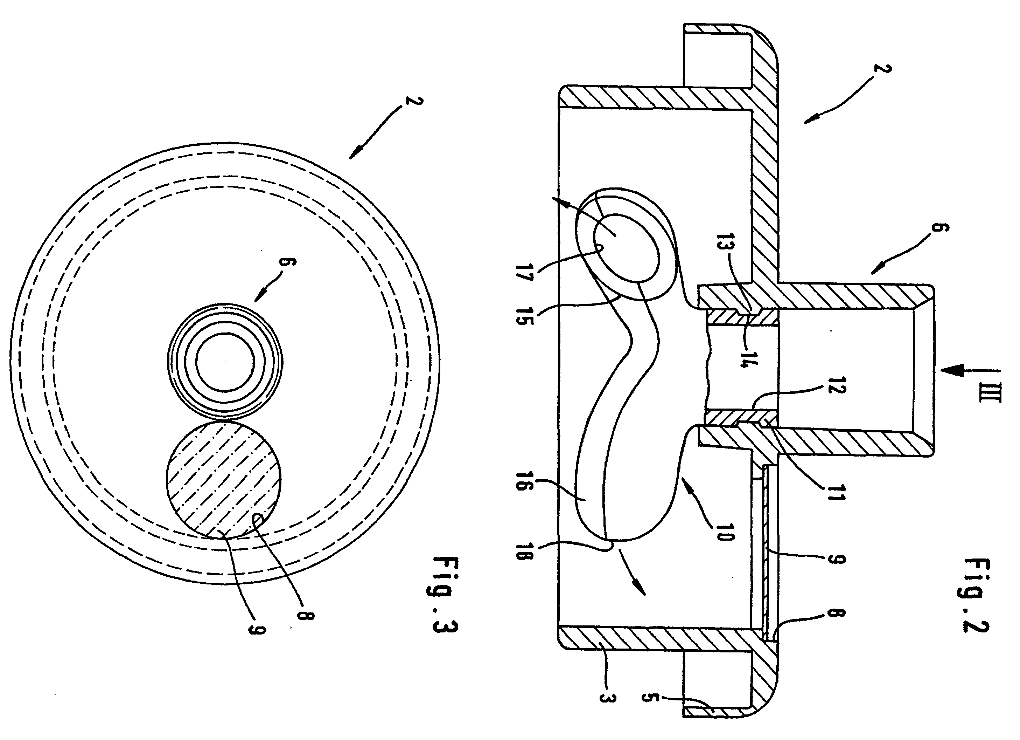

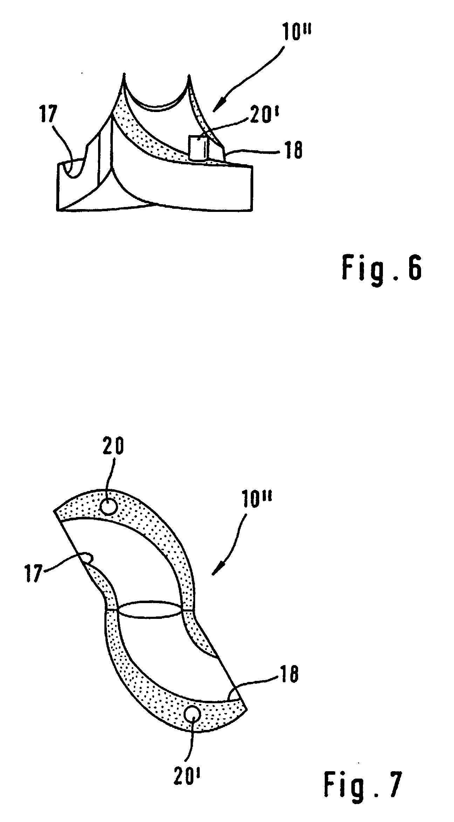

Im Zentrum des Deckels 2 ist ein Einlaufstutzen 6 zum Anschluß einer nicht dargestellten Zuführleitung und am unteren Ende der Kammer 1 ist ein Auslaufstutzen 7 zum Anschluß einer nicht dargestellten Ablaufleitung angeordnet, die jeweils einen Innendurchmesser aufweisen, der zusammen mit dem Durchmesser des einzufügenden Anschlußschlauches eine Preßpassung bildet. Der Einlaufstutzen 6 ist einstückig mit dem Deckel 2 ausgebildet. Neben dem Einlaufstutzten 6 ist der Deckel 2 kreisförmig ausgeschnitten. Die kreisförmige Öffnung 8 im Deckel 2, die sich von dem Einlaufstutzen 6 bis zur Wand 4 der Kammer 1 erstreckt, ist von einer kreisförmigen hydrophoben Membran 9 flüssigkeitsdicht verschlossen (Figuren 2 und 3). Stromabwärts des Einlaufstutzens 6 ist ein Strömungsleitbauteil 10 angeordnet, das im folgenden unter Bezugnahme auf die Figuren 2 bis 7 im einzelnen beschrieben wird.

Das Strömungsleitbauteil 10 weist ein kurzes in Längsrichtung der Kammer 1 angeordnetes Anschlußstück 11 mit einem zentralen Strömungskanal 12 auf, das passend in den Einlaufstutzten 6 des Deckels 2 eingesetzt ist. Der Einlaufstutzen 6 ist an eine Innenseite mit einem umlaufenden Ansatz 13 versehen, der in eine umlaufende Nut 14 an dem Anschlußstück 11 des Strömmungsleitbauteils 10 eingreift. Das Anschlußstück 11 geht in zwei Stömungsleitrohre 15, 16 über, die einstückiger Bestandteil des Anschlußstücks 11 und bezügl. der Längsachse der Kammer symmetrisch ausgebildet sind. Zur Verdeutlichung zeigt Figur 2 das zentrale Anschlußstück 11 in geschnittener Darstellung, während die beiden Strömungsleitrohre 15, 16, d.h. der untere Teil des Strömungsleitbauteils 10, in perspektivischer Darstellung dargestellt sind. Die Strömungsleitrohre 15, 16 erstrecken sich ausgehend von dem in Längsrichtung der Kammer angeordneten zentralen Anschlußstück 11 nach beiden Seiten in einer Raumkurve schraubenförmig in eine im wesentlichen tangential zur Wandung der Kammer 1 verlaufende horizontale Richtung. Die Öffnungen 17, 18 der beiden flügelartig abstehenden Strömungsleitrohre 15, 16 weisen dabei in entgegengesetzte Richtungen, wobei eine der beiden Öffnungen 18 dicht unterhalb der mit der Membran 9 verschlossenen Öffnung 8 im Deckel 2 liegt. Die Strömungsleitrohre 15, 16 weisen über ihre Länge einen im wesentlichen gleichbleibenden Kanalquerschnitt auf, der etwa halb so groß wie der Kanalquerschnitt des zentralen Anschlußstücks 11 ist. Die beiden Öffnungen 17, 18 der Strömungsleitrohre 15, 16 sind unmittelbar unterhalb der Unterseite des Deckels 2 angeordnet. Der Abstand zwischen der Unterseite des Deckels 2 und dem oberen Rand der Austrittsöffnungen 17, 18 entspricht etwa dem Außendurchmesser der Strömungsleitrohre 15, 16.

Während des Betriebs ist die Kammer vollständig mit Blut gefüllt. Durch die besondere Ausbildung des Strömungsleitbauteils 10 wird das durch den Einlaufstutzen 6 in das zentrale Anschlußstück 11 des Strömungsleitbauteils einströmende Blut in den beiden Strömungsleitrohren 15, 16 radial nach außen geleitet und dabei zusätzlich so umgelenkt, daß das Blut beim Austritt aus den Strömungsleitrohren in einer im wesentlichen tangential zur Wandung der Kammer verlaufenden horizontalen Richtung strömt. Die vertikale Strömung wird also in eine horizontale Zirkulationsströmung umgelenkt. Hierdurch wird eine schraubenlinienförmige Strömung induziert, wobei die kreisförmigen Anteile eine Druckunterschied aufbauen, der dazu führt, daß die Luftblasen in Richtung Längsachse gedrängt werden und aufgrund ihrer geringeren Dichte nach oben aufsteigen und durch die hydrophobe Membran 9 aus der Kammer 1 austreten. Da sich unmittelbar unterhalb des Deckels der Kammer eine kreisförmige Strömung ausbildet, wird die hydrophobe Membran 9 mit Blut umspült, und es treten keine Totzonen auf, in denen sich Luftblasen sammeln können. Aufgrund der starken Flüssigkeitsströmung entlang der Membran behält diese ihre Luftdurchlässigkeit auch nach mehrstündigem Gebrauch bei.

Nachdem das Blut von oben nach unten in schraubenlinienförmigen Bahnen durch die Kammer geflossen ist, fließt es durch eine in Figur 1 mit dem Bezugszeichen 19 versehene Filterkerze und dann durch den Auslaufstutzen 7 zur weiteren Verwendung ab. Es ist aber auch möglich, daß nur der Einlaufstutzen 6 in Kammerlängsrichtung angeordnet ist und der Auslaufstutzen 7 in tangentialer Richtung an die Kammer angeschlossen ist.

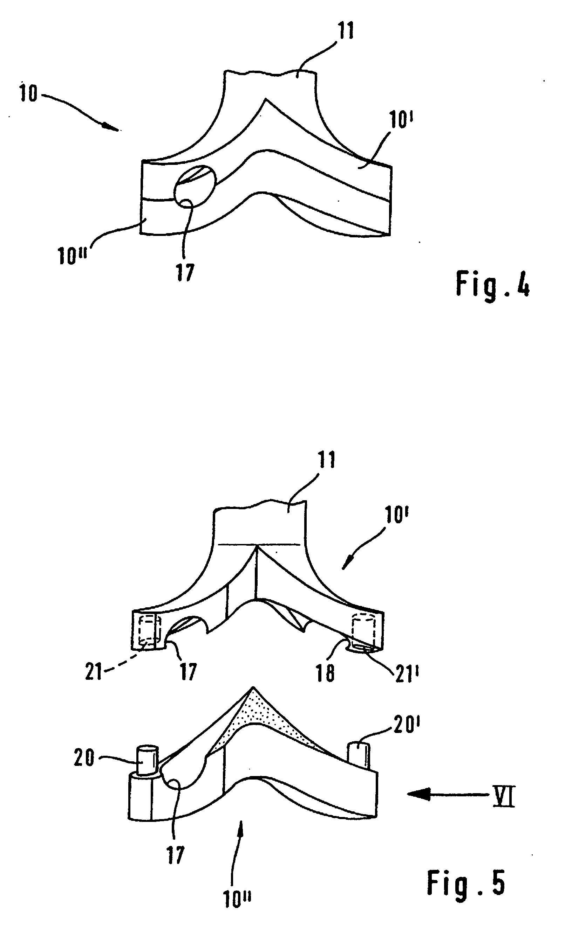

Aus fertigungstechnischen Gründen besteht das Strömungsleitbauteil 10 aus zwei Teilstücken 10', 10", die in Figur 4 dargestellt sind. Das obere Teilstück 10' umfaßt das zentrale Anschlußstück 11 und die obere Hälfte der beiden Strömungsleitrohre 15, 16, während das untere Teilstück 10" die untere Hälfte der beiden Strömungsleitrohre 15, 16 umfaßt. Damit sich die beiden Teilstücke 10', 10" nach der Fertigung einrasten und zusammensetzen lassen, sind an der unteren Hälfte zwei Stifte 20, 20' vorgesehen, die in entsprechende Ausnehmungen 21, 21' an dem oberen Teilstück 10' greifen und beide Teile fest zusammenhalten (Figur 5).

Sämtliche Teile des Luftabscheiders werden vorzugsweise im Spritzgußverfahren aus durchsichtigem Kunststoff hergestellt, so daß eine optische Kontrolle des Füllstandes und des Strömungsverlaufs jeder Zeit möglich ist. Die Ausbildung des Strömungsleitbauteils schafft einen Luftabscheider, der sich aufgrund einer möglichen koaxialen Anordnung von Einlauf- und Auslaufstutzen auf einfache Weise in ein bestehendes Schlauchsystem einfügen läßt und erlaubt eine Fertigung in großen Stückzahlen mit nur geringem technischen Aufwand.

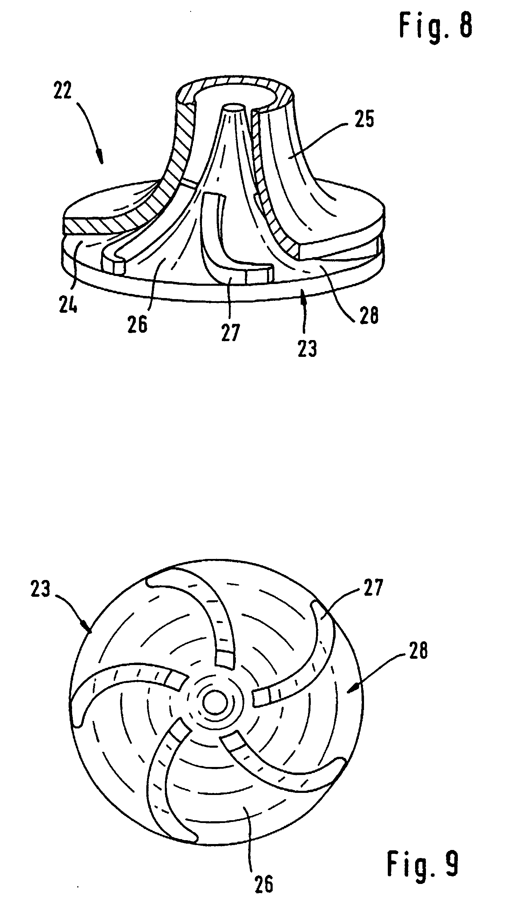

Figur 8 zeigt eine weitere Ausführungsform des Strömungsleitbauteils, das unmittelbar unterhalb des Deckels 2 der Kammer 1 an den in Figur 8 nicht dargestellten Einlaufstutzen 6 angeschlossen ist. Das Strömungsleitbauteil 22 weist einen rotationssymmetrischen Grundkörper 23 auf, dessen der einströmenden Flüssigkeit zugewandte Oberfläche geometrisch durch Rotation eines konkaven Kurvenabschnitts um die Längsachse des Einlaufstutzens 6 bzw. der Kammer 1 definiert ist. Unter Ausbildung eines schmalen Spaltes 24 ist oberhalb des Grundkörpers 23 ein zweiter rotationssymmetrischer Körper 25 angeordnet, der der konkaven Krümmung der Leitfläche 26 des Grundkörpers 23 folgt. Zwischen den beiden Rotationskörpern 23 und 25 verlaufen mehrere Leitschaufeln 27, die in Ebenen gekrümmt sind, die senkrecht auf der Längsachse der Rotationskörper stehen. Die Leitschaufeln 27 unterteilen den ringförmigen Spalt 24 in mehrere Strömungskanäle 28, die sich in einer Raumkurve aus der Kammerlängsrichtung in eine im wesentlichen tangential zur Wandung der Kammer verlaufende Richtung erstrecken. Da die Austrittsöffnungen der Strömungskanäle 28 unmittelbar unterhalb des Deckels 2 angeordnet sind, bildet sich eine kreisförmige Strömung unterhalb des Deckels aus, so daß die Membran 9 mit Blut umspült wird. Diese kreisförmige Strömung überlagert sich mit der in Längsrichtung von dem Einlaufstutzen 6 zu dem Auslaufstutzen 7 gerichteten Strömung. Während das Blut durch die Kammer fließt, werden die Luftblasen abgeschieden und über die hydrophobe Membran 9 aus der Kammer 1 entfernt.

## Patentansprüche

1. Vorrichtung zum Abscheiden von Gasblasen aus Flüssigkeiten, insbesondere Blut, mit einer im wesentlichen zylinderförmigen Kammer (1), einem in Längsrichtung der Kammer (1) angeordneten Einlaufstutzen (6), einem Auslaufstutzen (7) und einem stromab des Einlaufstutzens (6) angeordneten Strömungsleitbauteil (10), das mindestens einen Strömungskanal aufweist, der sich in einer Raumkurve aus der Kammerlängsrichtung in eine im wesentlichen tangential zur Wandung der Kammer verlaufende Richtung erstreckt, **dadurch gekennzeichnet,**
**daß** im Deckelteil (2) der Kammer (1) mindestens eine mit einer hydrophoben Membran (9) verschlossene Öffnung (8) vorgesehen ist, wobei der im wesentlichen tangential zur Wandung (4) der Kammer (1) verlaufende Abschnitt des mindestens einen Strömungskanals derart unmittelbar unterhalb des Deckelteils (2) angeordnet ist, daß die Membran (9) von der einströmenden Flüssigkeit unter Vermeidung der Ausbildung von Totzonen umspülbar ist.

2. Vorrichtung nach Anspruch 1, **dadurch gekennzeichnet, daß** das Strömungsleitbauteil (10) ein an den Einlaufstutzen (6) angeschlossenes rohrförmiges Anschlußstück (11) aufweist, das in mindestens ein Strömungsleitrohr (15,16) übergeht, das sich in einer Raumkurve aus der Kammerlängsrichtung in eine im wesentlichen tangential zur Wandung (4) der Kammer (1) laufende Richtung erstreckt.

3. Vorrichtung nach Anspruch 2, **dadurch gekennzeichnet, daß** das Strömungsleitbauteil (10) zwei Strömungsleitrohre (15,16) aufweist, deren Austrittsöffnungen (17,18) in entgegengesetzte Richtungen zeigen.

4. Vorrichtung nach Anspruch 3, **dadurch gekennzeichnet, daß** das erste und zweite Strömungsleitrohr (15,16) bezügl. der Längsachse des Einlaufstutzens (6) symmetrisch sind.

5. Vorrichtung nach einem der Ansprüche 1 bis 4, **dadurch gekennzeichnet, daß** die mit der hydrophoben Membran (9) verschlossene Öffnung (8) kreisförmig ist.

6. Vorrichtung nach Anspruch 6, **dadurch gekennzeichnet, daß** die mit der hydrophoben Membran (9) verschlossene Öffnung im Deckelteil (2) neben dem Einlaufstutzen (6) angeordnet ist.

7. Vorrichtung nach Anspruch 6, **dadurch gekennzeichnet, daß** sich die mit der hydrophoben Membran (9) verschlossene Öffnung (8) zwischen dem Einlaufstutzen (6) und der Wand (4) der Kammer (1) erstreckt.

8. Vorrichtung nach einem der Ansprüche 1 bis 7, **dadurch gekennzeichnet, daß** das Strömungsleitbauteil (22) einen Grundkörper (23) aufweist, dessen der einströmenden Flüssigkeit zugewandte Oberfläche durch Rotation eines Kurvenabschnitts um die Längsachse der Kammer (1) definiert ist, wobei auf der Oberfläche des Grundkörpers (23) Leitschaufeln (27) angeordnet sind, die in auf der Rotationsachse senkrecht stehenden Ebenen gekrümmt sind.

9. Vorrichtung nach Anspruch 8, **dadurch gekennzeichnet, daß** das Strömungsleitbauteil (22) einen zweiten rotationssymmetrischen Körper (25) aufweist, der unter Ausbildung eines ringförmigen Spaltes (24) stromauf des Grundkörpers angeordnet ist.

10. Vorrichtung nach Anspruch 9, **dadurch gekennzeichnet, daß** die Höhe der Leitschaufeln (27) derart bemessen ist, daß sich diese bis zu dem zweiten rotationssymmetrischen Körper (25) des Strömungsleitbauteils (22) erstrecken.

## Claims

1. Device for separating gas bubbles from liquids, particularly blood, with an essentially cylindrical chamber (1), and inlet connector (6) arranged in the longitudinal direction of the chamber (1), and outlet connector (7) and a flow guidance device (10) arranged downstream of the inlet connector (6), that has at least one flow channel extending in a space curve from the chamber longitudinal direction in a direction running essentially tangential to the wall of the chamber, **characterised in that**
in the cover section (2) of the chamber (1) at least one opening (8) closed by a hydrophobic membrane (9) is provided, whereby the section of at least one flow channel that runs essentially tangential to the wall (4) of the chamber (1) is arranged immediately below the cover part (2) in such a way that membrane (9) is flushed by the inflowing liquid with the formation of dead zones being avoided.

2. Device in accordance with claim 1, **characterised in that** the flow guidance unit (10) has a tubular connection piece (11) connected to the inlet connector (6), that merges into at least one flow guidance tube (15, 16) that extends in a space curve from the longitudinal direction of the chamber in a direction running essentially tangential to the wall (4) of the chamber (1).

3. Device in accordance with claim 2, **characterised in that** the flow guidance unit (10) has two flow guidance tubes (15, 16), whose outlet openings (17, 18) point in opposite directions.

4. Device in accordance with claim 3, **characterised in that** the first and second flow guidance tubes (15, 16) are symmetrical relative to the longitudinal axis of the inlet connector (6).

5. Device in accordance with one of claims 1 to 4, **characterised in that** the opening (8) closed by the hydrophobic membrane (9) is circular.

6. Device in accordance with claim 6, **characterised in that** the opening in the cover part (2) closed by the hydrophobic membrane (9) is arranged next to the inlet connector (6).

7. Device in accordance with claim 6, **characterised in that** the opening (8) closed by the hydrophobic membrane (9) extends between the inlet connector (6) and the wall (4) of the chamber (1).

8. Device in accordance with one of claims 1 to 7, **characterised in that** the flow guidance unit (22) has a base body (23) whose surface facing towards the inflowing liquid is defined by rotation of a curve section around the longitudinal axis of the chamber (1), with guide vanes (27) curved in a plane vertical to the axis of rotation being arranged on the surface of the base body (23).

9. Device in accordance with claim 8, **characterised in that** the flow guidance unit (22) has a second dynamically-balanced body (25) that is arranged upstream of the base body, with an annular gap (24) being formed.

10. Device in accordance with claim 9, **characterised in that** the height of the guide vanes (27) is dimensioned so that they extend to the second dynamically-balanced body (25) of the flow guidance unit (22).

## Revendications

1. Dispositif pour l'extraction de bulles de gaz contenues dans des liquides, en particulier le sang, avec une chambre (1) de forme sensiblement cylindrique, une tubulure d'entrée (6) disposée dans la direction longitudinale de la chambre (1), une tubulure de sortie (7) et un élément de canalisation de l'écoulement (10) disposé en aval de la tubulure d'entrée (6), lequel comprend au moins un canal d'écoulement qui s'étend selon une courbe spatiale hors de la direction longitudinale de la chambre dans une direction sensiblement tangentielle à la paroi de la chambre, **caractérisé en ce qu'**au moins une ouverture (8) fermée par une membrane hydrophobe (9) est prévue dans le couvercle (2) de la chambre (1), de manière que la section d'au moins un canal d'écoulement s'étendant sensiblement tangentiellement à la paroi (4) de la chambre (1) soit disposée immédiatement sous le couvercle (2), de telle manière que la membrane (9) soit investie par le fluide affluant en évitant la formation de zones mortes.

2. Dispositif selon la revendication 1, **caractérisé en ce que** l'élément de canalisation de l'écoulement (10) comporte une partie de jonction tubulaire (11) raccordée à la tubulure d'entrée (6), laquelle débouche dans au moins un conduit de canalisation d'écoulement (15,16) qui s'étend selon une courbe spatiale hors de la direction longitudinale de la chambre dans une direction sensiblement tangentielle à la paroi de la chambre (1).

3. Dispositif selon la revendication 2, **caractérisé en ce que** l'élément de canalisation de l'écoulement (10) comporte deux conduits de canalisation de l'écoulement (15,16) dont les orifices de sortie (17, 18) sont dirigés dans des directions opposées.

4. Dispositif selon la revendication 3, **caractérisé en ce que** les premier et deuxième conduits de canalisation de l'écoulement (15,16) sont symétriques par rapport à l'axe longitudinal de la tubulure d'entrée (6).

5. Dispositif selon une des revendications 1 à 4, **caractérisé en ce que** l'ouverture (8) fermée par la membrane hydrophobe (9) est circulaire.

6. Dispositif selon la revendication 6, **caractérisé en ce que** l'ouverture fermée par la membrane hydrophobe (9) est située dans le couvercle (2) à côté de la tubulure d'entrée (6).

7. Dispositif selon la revendication 6, **caractérisé en ce que** l'ouverture (8) fermée par la membrane hydrophobe (9) s'étend entre la tubulure d'entrée (6) et la paroi (4) de la chambre (1).

8. Dispositif selon une des revendications 1 à 7, **caractérisé en ce que** l'élément de canalisation de l'écoulement (22) comprend une base (23) dont la surface orientée vers le fluide affluant est délimitée par la rotation d'une section courbe autour de l'axe longitudinal de la chambre (1), et où des aubes directrices (27) soit disposées sur la surface de la base (23), lesquelles sont courbées dans un plan perpendiculaire à l'axe de rotation.

9. Dispositif selon la revendication 8, **caractérisé en ce que** l'élément de canalisation de l'écoulement (22) comporte un deuxième corps (25) à symétrie de révolution qui est disposé en amont de la base en formant un interstice annulaire (24).

10. Dispositif selon la revendication 9, **caractérisé en ce que** la hauteur des aubes directrices (27) est dimensionnée de telle manière que celles-ci s'étendent jusqu'au deuxième corps (25) à symétrie variable de l'élément de canalisation de l'écoulement (22).
